# EUROPEAN PATENT SPECIFICATION

(11) **EP 1 232 760 B1**
(45) Date of publication and mention of the grant of the patent: **05.09.2007**
(21) Application number: 01830108.5
(22) Date of filing: 16.02.2001
(51) Int. Cl.: A61L 2/08, B65B 55/08, A23L 3/26

(54) **Method and unit for sterilizing packaging sheet material for manufacturing sealed packages of pourable food products**
Verfahren und Anlage zur Sterilisierung von flächigem Verpackungsmaterial zur Herstellung von verschlossenen Verpackungen von fliessfähigen Nahrungsmitteln
Procédé et installation pour la sterilisation de matériaux d'emballage en feuille pour la fabrication d'emballages hermétiques de produits alimentaires liquides

(43) Date of publication of application: 21.08.2002
(73) Proprietor: Tetra Laval Holdings & Finance SA, 1009 Pully (CH)
(72) Inventor: Möller, Hakan, 22361 Lund (SE); Näslund, Lars, 244 02 Furulund (SE); Schianchi, Roberto, 211 42 Malmö (SE)
(74) Representative: Franzolin, Luigi

(56) References cited:
- EP-A- 0 622 979
- WO-A-98/18608
- FR-A- 2 773 714
- GB-A- 1 460 134
- DATABASE WPI Section Ch, Week 199923 Derwent Publications Ltd., London, GB; Class D14, AN 1999-270508 XP002170154 & JP 11 084100 A (NISSIN HIGH VOLTAGE KK), 26 March 1999 (1999-03-26)
- DATABASE WPI Section Ch, Week 199731 Derwent Publications Ltd., London, GB; Class D14, AN 1997-340481 XP002170155 & SE 9 503 810 A (TETRA LAVAL HOLDINGS & FINANCE SA), 1 May 1997 (1997-05-01)

## Description

The present invention relates to a method and unit for sterilizing packaging sheet material for manufacturing sealed packages of pourable food products.

As is known, numerous pourable food products, such as fruit juice, UHT (ultra-high-temperature processed) milk, wine, tomato sauce, etc., are sold in packages formed, on fully automatic packaging machines, from packaging sheet material, which may be constituted by pre-cut blanks or by a continuous web or strip of packaging material which is folded and longitudinally sealed to define a continuous, longitudinally sealed packaging material tube.

The packaging material may be constituted by a single or multi-layer plastics material, polymeric material, mineral-filled polymeric material or a laminated paperboard-type material. By way of example, one known type of paperboard-type packaging material has a multilayer structure comprising a layer of paper material covered on both sides with layers of heat-seal material, e.g. polyethylene. In the case of aseptic packages for long-storage products such as UHT milk, the packaging sheet material comprises a layer of barrier material defined, for example, by an aluminium film, which is superimposed on a layer of heat-seal plastic material and is in turn covered with another layer of heat-seal plastic material which eventually defines the inner face of the package contacting the food product.

For producing aseptic packages in conventional form, fill and seal-type packaging machines, the strip of packaging material is provided, e.g. unwound off a reel, and fed through a sterilizing unit in which it is sterilized, for example, by immersion in a bath of liquid sterilizing agent such as a concentrated solution of hydrogen peroxide and water.

More specifically, the sterilizing unit comprises a bath filled, in use, with the sterilizing agent in which the strip is fed. The bath conveniently comprises two parallel vertical branches connected at the bottom to define a U-shaped path of a length depending on the traveling speed of the strip and such as to allow enough time to treat the packaging material. For effective, fairly fast treatment, so as to reduce the size of the sterilizing chamber, the sterilizing agent must be maintained at a high temperature of, say, roughly 70°C.

The sterilizing unit also comprises an aseptic chamber in which the strip of packaging material issuing from the sterilizing bath is treated mechanically (e.g. by means of drying rollers) and thermofluidically (e.g. by means of hot-air jets) to remove any residual sterilizing agent. The amount of residual sterilizing agent allowed in the packaged product, in fact, is governed by strict standards (the maximum permissible amount being in the order of a 0.5 parts per million). The aseptic chamber must also be maintained slightly above ambient pressure to ensure any leakage through the seals occurs outwards as opposed to inwards of the chamber, to keep out any contaminating agents.

Before leaving the aseptic chamber, the strip is folded into a cylinder and sealed longitudinally to form in known manner a continuous, vertical, longitudinally sealed tube. The tube of packaging material, in fact, forms an extension of the aseptic chamber and is filled continuously with the pourable product and then fed to a forming unit for forming individual packages and by which the tube is gripped between pairs of jaws to seal the tube transversely and form aseptic pillow packs.

The pillow packs are separated by cutting the sealed portions between the packs, and are then fed to a final folding station where they are folded mechanically into the finished form.

Alternatively, the packaging material is sterilized by applying, on the side of the packaging material eventually defining the inner face of the package, a thin film of hydrogen peroxide, which is later removed by heating. Sterilizing units are also known in which the hydrogen peroxide is applied to the surface of the packaging sheet material by liquid atomization or gas condensation.

Packaging machines of the above type are used widely and satisfactorily in a wide range of food industries; and performance of the sterilizing unit, in particular, is such as to amply conform with standards governing asepticity of the packages and residual sterilizing agent.

Within the industry, however, demand for further improvement exists, especially as regards elimination of residual sterilizing agent, and which stems, in particular, from market demand for packages featuring reclosable opening devices which are easy to open and provide for easy pouring of the product.

In the case of non-aseptic packaging machines, such devices can be applied, e.g. injection molded directly, to the strip material before the packages are formed.

Conversely, in the case of aseptic packaging machines, any opening devices are normally applied after the packages are formed, which poses drawbacks from the production standpoint by requiring the use of sophisticated systems for supplying and applying the devices.

The above mentioned opening devices, if applied beforehand to the packaging sheet material, form breaks in the geometric continuity of the packaging sheet material, in which residual sterilizing agent may become trapped, and from which the sterilizing agent cannot be removed completely using known techniques.

On the other hand, using additional means to remove the sterilizing agent may have a negative effect on the operating parameters, in particular temperature and pressure, of the aseptic chamber, and impair performance of the sterilizing unit as a whole.

To eliminate the above drawbacks, sterilizing units have been devised using electron beams to irradiate moving sheet packaging material.

More specifically, as known from SE-A-9503810, the sheet packaging material is irradiated on one face by an electron beam issuing from an accelerator located to one side of the packaging material.

The electron beam penetrates the packaging material and so sterilizes both opposite faces simultaneously.

The above method, however, is not without drawbacks of its own, which mainly lie in alteration of the packaging material by passage of the electrons. More specifically, the packed product can acquire, from te packaging material, an unpleasant, so-called "off-flavour".

Furthermore, the preambles of independent claims 1 and 8 are known from document EP-A-0 622 979.

It is an object of the present invention to provide a method of sterilizing sheet packaging material for producing sealed packages of pourable food products, designed to provide an effective, straightforward, low-cost solution to the aforementioned problems.

The present invention is directed to a method and a unit for sterilizing sheet packaging material as defined in independent claims 1 and 8.

A preferred, non-limiting embodiment of the present invention will be described by way of example with reference to the accompanying drawings, in which:
Figure 1 shows a lateral elevation of a portion of a packaging machine for producing aseptic sealed packages of pourable food products and featuring a sterilizing unit in accordance with the present invention;
Figures 2 shows an enlarged view in perspective of the Figure 1 sterilizing unit;
Figure 3 shows a larger-scale section of a detail of the Figure 1 sterilizing unit; and
Figure 4 shows a section of a portion of a packaging sheet material for manufacturing packages of pourable food products.

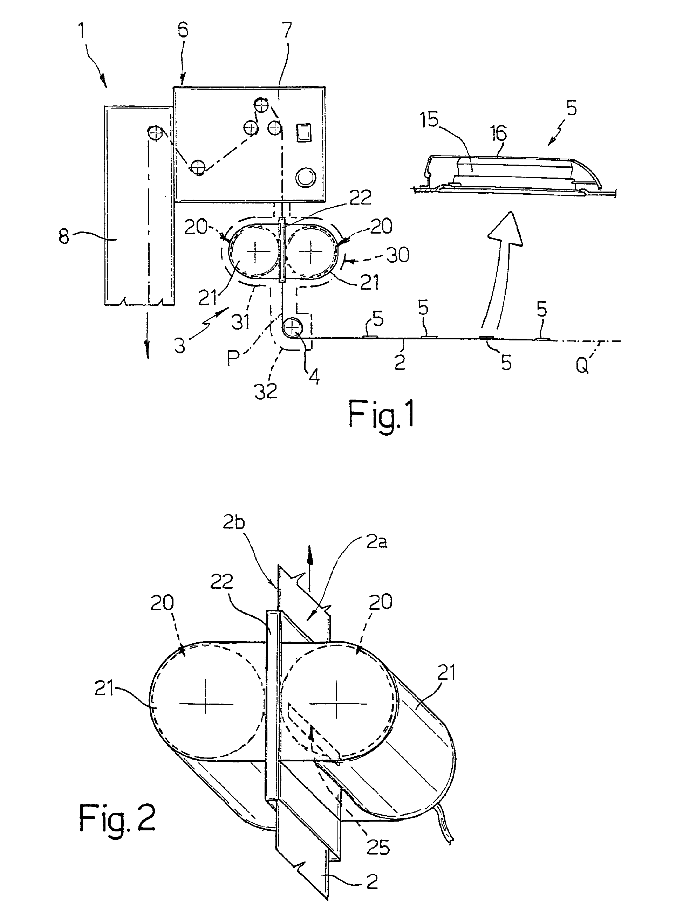

Number 1 in Figure 1 indicates as a whole a packaging machine for producing aseptic sealed packages (not shown) of pourable food products from a packaging sheet material 2.

Machine 1 comprises a sterilizing unit 3 for sterilizing the packaging sheet material 2, which is fed, for example, off a known reel (not shown) along a substantially vertical path P. More specifically, on leaving the reel, packaging sheet material 2 is fed, in the illustrated example, along a substantially horizontal path Q and is detoured along vertical path P by a guide roller 4.

To produce packages with pre-applied auxiliary items, such as reclosable opening devices 5 of plastic material - e.g. of the type illustrated in International Patent Application WO 98/18684 filed by the present Applicant - packaging sheet material 2 is fed through a conventional application unit (not shown) - e.g. an injection-molding unit of the type described in International Patent Application WO 98/18608 filed by the present Applicant - at the output of which packaging sheet material 2 comprises a succession of opening devices 5 equally spaced along an intermediate longitudinal portion of packaging sheet material 2. At the output of the application unit and upstream from sterilizing unit 3, a store (not shown) is conveniently provided to store packaging sheet material 2 and compensate for the different feeds of the two units (step feed and continuous feed respectively).

Machine 1 also comprises a chamber 6 in which packaging sheet material 2 is kept in a sterile-air environment. Chamber 6 comprises a top portion 7, from which sterilizing unit 3 extends downwards; and a bottom portion or tower 8, which extends vertically on one side of top portion 7 and parallel to sterilizing unit 3, and wherein the packaging sheet material, which in the illustrated example comprises a web or strip 2, is folded longitudinally into a cylinder and sealed longitudinally to form a continuous tube (not shown). The tube is filled continuously with the sterilized or sterile-processed food product for packaging, is sealed along equally spaced cross sections, and is subjected to successive mechanical folding operations to form the finished packages.

It should be noted that the sterilizing unit 3 may be located at any convenient position with respect to chamber 6 or even incorporated therein.

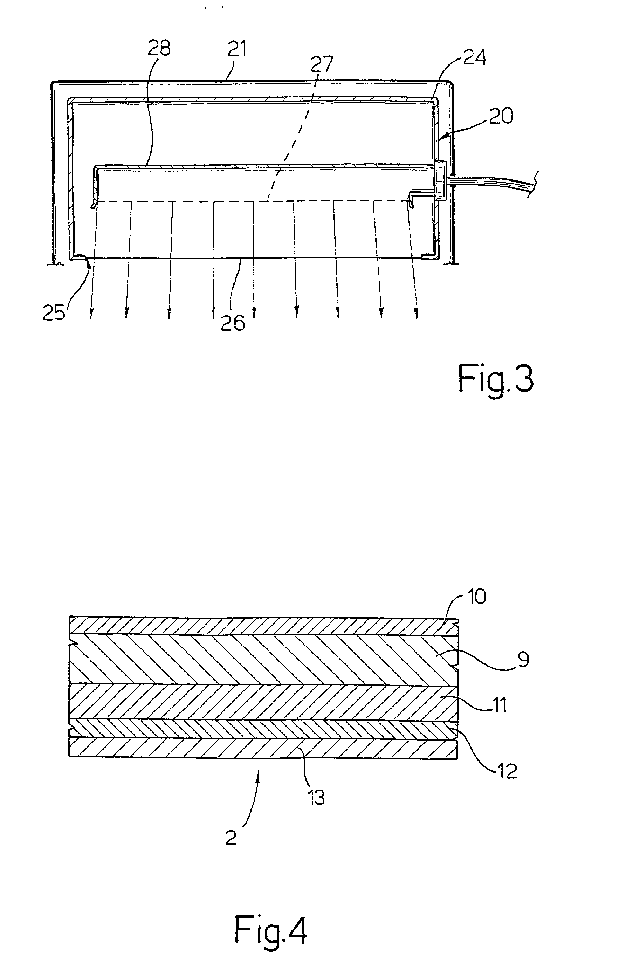

With reference to Figure 4, the packaging sheet material 2, shown by way of example, has a multilayer structure and substantially comprises a layer of fibrous material 9, e.g. paper, covered on both sides with respective layers 10, 11 of heat-seal plastic material, e.g. polyethylene. Being used to produce aseptic packages of long-storage products, such as UHT milk, packaging sheet material 2 also comprises, on the side eventually contacting the food product, a layer of oxygen and light barrier material defined, for example, by a sheet of aluminium 12, which in turn is covered with a further layer 13 of heat-seal plastic material, e.g. polyethylene. As mentioned hereinabove, the packaging sheet material may also be constituted by a single or multi-layer plastics material, polymeric material, mineral-filled polymers, etc.

Each opening device 5 (Figure 1) comprises in known manner a base portion 15 fixed to packaging sheet material 2; and a cap portion 16 hinged laterally to and closed on base portion 15.

An important aspect of the present invention is that sterilizing unit 3 comprises two known electron irradiation sources 20 located on opposite sides of packaging sheet material 2 and simultaneously activated for directing respective electron beams, having an energy at most equal to 100 KeV, onto opposite faces 2a, 2b of the packaging sheet material 2.

In the example shown, irradiation sources 20 are aligned with each other in a direction perpendicular to sheet packaging material 2. Alternatively, irradiation sources 20 may even be offset with respect to each other.

The irradiation sources 20 are preferably incorporated in respective fixed housings 21, between which packaging sheet material 2 to be sterilized, and already fitted with auxiliary items, e.g. opening devices 5, is fed.

With particular reference to Figures 1 and 2, housings 21 are fixed to the structure of packaging machine 1 and cooperate on opposite sides with a thin, box-shaped guide member 22 fixed between supporting members 21 and through which, in use, packaging sheet material 2 for sterilizing is fed.

Each irradiation source 20 comprises a tubular enclosure 24 having an axis parallel to the plane of the packaging sheet material 2 and perpendicular to path P, and which is kept under vacuum and has an aperture 25 facing material 2 and closed by a window foil 26 which is easily penetrated by electrons. Window foil 26 is constituted, for example, by a sheet of material such as titanium, aluminium, silicon, etc., having a thickness of a few µm, e.g., 2-35 µm, and preferably 2-8 µm.

Each irradiation source 20 also comprises an electron-emitting member such as, e.g., a tungsten filament 27 (shown schematically by the dash line in Figure 3), which, in the illustrated example, is housed inside a casing 28 in turn fitted inside enclosure 24, and is heated to emit electrons. Any other electron emitting means may be used.

In use, the electrons are accelerated, in the form of a beam between the filament 27 at negative potential and the window foil 26 which is at ground potential.

In particular, the electrons emitted are vacuum accelerated into beams directed on to sheet packaging material 2 by respective electric fields generated by potential differences of 90 kV or less and preferably less than 80 kV.

The electrons reach their maximum speed inside the vacuum environment defined by enclosure 24, and decelerate and gradually lose part of their energy on colliding with the atoms constituting window foil 26 and sheet packaging material 2.

In the example shown, the energy produced by the electron beams striking sheet packaging material 2 kills any microorganisms in the material.

Given their low energy level (100 KeV), the electron beams generated by irradiation sources 20 penetrate sheet packaging material 2 to a depth of a few µm, which is sufficient to ensure surface sterilization of sheet packaging material 2 on both faces 2a, 2b, while at the same time minimizing the effect of radiation on the packaging material itself.

Unit 3 also comprises a shielding member 30 (indicated by the dash line in Figure 1) fixed to the structure of packaging machine 1 and in turn comprising a first portion 31 surrounding housings 21 of electron irradiation sources 20, and a substantially L-shaped second portion 32 extending downwards from portion 31 and enclosing guide roller 4 and the portion of packaging sheet material 2 extending between roller 4 and electron irradiation sources 20. Portions 31, 32 are all shielding but other solutions are possible.

Using electron acceleration potentials of less than 90 kV, shielding member 30 may be made indifferently of steel of less than 20 mm thickness - e.g. 12 mm with an acceleration potential Va of 75 kV - or of lead of unit mm thickness - e.g. 2 mm with an acceleration potential Va of 75 kV, or 1 mm with an acceleration potential Va of 50 kV. Tests have shown shielding members 30 formed as described above to be capable of protecting the area around irradiation sources 20 from X-rays produced as a secondary effect of electron absorption by packaging sheet material 2 or parts of packaging machine 1.

The advantages of sterilizing unit 3 according to the present invention will be clear from the foregoing description.

In particular, by using two irradiation sources 20 generating respective low-voltage electron beams directed on to opposite faces 2a, 2b of sheet packaging material 2 and having an energy of at most 100 KeV, each beam penetrates the packaging material to a depth of a few µm, which, as stated, is sufficient to ensure surface sterilization of both opposite faces of the packaging material, while at the same time minimizing any possible alteration of the packaging material, and so preventing the packaging material from acquiring an unpleasant taste which may be transmitted to the food product.

Furthermore, using electron beams, the sterilizing system described leaves no residue on the processed materials, and may therefore be used to sterilize packaging sheet material (2) with items applied thereto such as preapplied opening devices (5). This affords enormous advantages in terms of production, by opening devices 5 being much easier and cheaper to apply directly to packaging sheet material 2 than to the finished packages. Moreover, no additional means are required for removing from the packaging material the sterilizing agent normally used in known units of the type described previously.

Sterilizing unit 3 is particularly effective, by the electron beams emitted being capable of reaching any surface or irregularity of opening devices 5.

Finally, the output capacity of sterilizing unit 3 may be increased easily with no alterations to the unit required.

Clearly, changes may be made to sterilizing unit 3 as described and illustrated herein without, however, departing from the scope of the accompanying Claims.

## Claims

1. A method of sterilizing sheet packaging material (2) for producing sealed packages of pourable food products, the method comprising the step of irradiating the packaging material (2) with electrons; said irradiating step comprising the step of directing on to opposite faces (2a, 2b) of said sheet packaging material (2) respective low-voltage electron beams, each having an energy of at most 100 KeV, **characterized in that** it comprises the step of aligning said electron beams with each other in a direction perpendicular to said packaging sheet material (2) and directing said electron beams simultaneously on to said opposite faces (2a, 2b) of said sheet packaging material to ensure simultaneous surface sterilization of both sides of said sheet packaging material (2).

2. A method as claimed in claim 1, wherein said step of directing causes that each said electron beam penetrates the surface of the packaging material only to a depth of few µm to minimize any alteration of the material that could result in an unpleasant taste transimitted to a food product packaged in said material; said packaging material being of the type having a multilayer structure and including, on a side thereof adapted to be placed in contact with a food product, a layer of oxygen and light barrier material (12) and a layer of heat-sealable plastic material (13).

3. A method as claimed in Claim 1 or 2, **characterized in that** said step of directing each said electron beam comprises the step of irradiating electrons and accelerating said electrons on said opposite faces (2a, 2b) of said sheet packaging material.

4. A method as claimed in any one of foregoing claims, **characterized in that** said step of accelerating said electrons is performed by applying an electric field generated by a potential difference of 90 kV or less.

5. A method as claimed in Claim 4, **characterized in that** said potential difference is less than 80 kV.

6. A method as claimed in any one of the foregoing Claims, **characterized by** being applied to opposite faces (2a, 2b) of a sheet packaging material (2) having a number of prefitted auxiliary members (5).

7. A method as claimed in any one of the foregoing Claims, **characterized by** being applied to opposite faces (2a, 2b) of a sheet packaging material (2) in the form of a continuous web.

8. A unit (3) for sterilizing sheet packaging material (2) for producing sealed packages of pourable food products, the unit comprising irradiation means (20) for irradiating said sheet packaging material (2) with electrons, said irradiation means comprising two generating devices (20) for generating low-voltage electron beams, and which are located on opposite sides of said sheet packaging material (2) and generate respective electron beams directed on to opposite faces (2a, 2b) of the packaging material (2) and each having an energy of at most 100 keV, **characterized in that** said generating devices (20) are aligned with each other in a direction perpendicular to said sheet packaging material (2) and simultaneously activated, for simultaneously irradiating opposite sides (2a, 2b) of a packaging material (2) to ensure simultaneous surface sterilization of both sides of said sheet packaging material (2).

9. A unit as claimed in claim 8, **characterized in that** said generating device (20) comprises electron emitting means (27) and generating means (27, 26) for generating an electric field and accelerating said electrons into a beam directed on to said sheet packaging material (2).

10. A unit as claimed in Claim 9, **characterized in that s**aid electric field is generated by a potential difference of 90 kV or less.

11. A unit as claimed in Claim 10, **characterized in that** said potential difference is less than 80 kV.

12. A unit as claimed in any one of Claims 8 to 11, **characterized by** comprising at least one shield (30) for shielding the region surrounding said generating devices (20) for generating electron beams.

13. A unit as claimed in Claim 12, **characterized in that** said shield (30) is made of steel of less than 20 mm in thickness.

14. A unit as claimed in Claim 13, **characterized in that** said shield (30) is made of lead of about 3 mm in thickness.

## Patentansprüche

1. Verfahren zur Sterilisierung von blattförmigem Verpackungsmaterial (2) zur Herstellung von versiegelten Verpackungen für schütt- bzw. fließfähige Nahrungsmittel, wobei das Verfahren den Schritt des Bestrahlens des Verpackungsmaterials (2) mit Elektronen umfasst; wobei der Schritt des Bestrahlens den Schritt des Richtens von entsprechenden Niederspannungselektronenstrahlen auf zwei gegenüberliegende Seiten (2a, 2b) des blattförmigen Verpackungsmaterials (2) umfasst, die jeweils eine Energie von höchstens 100 KeV aufweisen, **dadurch gekennzeichnet, dass** es den Schritt des zueinander Ausrichtens der Elektronenstrahlen in eine Richtung senkrecht zum blattförmigen Verpackungsmaterial (2) sowie den des Richtens der Elektronenstrahlen gleichzeitig auf zwei gegenüberliegende Seiten (2a, 2b) des Verpackungsmaterials umfasst, um die gleichzeitige Sterilisierung beider Seiten des blattförmigen Verpackungsmaterials (2) zu gewährleisten.

2. Verfahren nach Anspruch 1, wobei der Schritt des Richtens bewirkt, dass jeder Elektronenstrahl die Oberfläche des Verpackungsmaterials nur bis zu einer Tiefe von wenigen µm durchdringt, um jegliche Änderung des Materials zu minimieren, die zur Übertragung eines unangenehmen Geschmacks auf ein in diesem Material verpacktes Nahrungsmittel führen könnte, wobei das Verpackungsmaterial zu der Sorte gehört, die eine mehrschichtige Struktur hat und auf einer Seite, die dazu ausgelegt ist, mit dem Nahrungsmittel in Berührung zu kommen, eine Schicht aus Sauerstoff- und Lichtsperrmaterial (12) sowie eine Schicht aus heißsiegelbarem Kunststoffmaterial (13) enthält.

3. Verfahren nach Anspruch 1 oder 2, **dadurch gekennzeichnet, dass** der Schritt des Richtens jedes Elektronenstrahls den Schritt des Bestrahlens von Elektronen und Beschleunigens der Elektronen auf den gegenüberliegenden Seiten (2a, 2b) des blattförmigen Verpackungsmaterials umfasst.

4. Verfahren nach einem der vorhergehenden Ansprüche, **dadurch gekennzeichnet, dass** der Schritt des Beschleunigens der Elektronen durch Anbringen eines elektrisches Felds, das durch einen Spannungsdifferenz von 90 kV oder weniger erzeugt wird, erfolgt.

5. Verfahren nach Anspruch 4, **dadurch gekennzeichnet, dass** die Spannungsdifferenz weniger als 80 kV beträgt.

6. Verfahren nach einem der vorhergehenden Ansprüche, **gekennzeichnet durch** dessen Anwendung auf zwei gegenüberliegende Seiten (2a, 2b) eines blattförmigen Verpackungsmaterials (2) mit einer Anzahl vorangebrachter Hilfsteile (5).

7. Verfahren nach einem der vorhergehenden Ansprüche, **gekennzeichnet durch** dessen Anwendung auf zwei gegenüberliegende Seiten (2a, 2b) eines blattförmigen Verpackungsmaterials (2) in Form einer kontinuierlichen Bahn.

8. Einheit (3) zur Sterilisierung von blattförmigen Verpackungsmaterial (2) zur Herstellung von versiegelten Verpackungen für schütt- bzw. fließfähige Nahrungsmittel, wobei die Einheit Bestrahlungsmittel (20) zum Bestrahlen des blattförmigen Verpackungsmaterials (2) mit Elektronen umfasst, wobei die Bestrahlungsmittel zwei Erzeugungsmittel (20) zum Erzeugen von Niederspannungselektronenstrahlen umfassen, und die auf gegenüberliegenden Seiten eines blattförmigen Verpackungsmaterials (2) angeordnet sind und entsprechende Elektronenstrahlen erzeugen, die auf die gegenüberliegenden Seiten (2a, 2b) des Verpackungsmaterials (2) gerichtet sind und die jeweils eine Energie von höchstens 100 keV haben, **dadurch gekennzeichnet, dass** die Erzeugungsmittel (20) zueinander in eine Richtung senkrecht zum blattförmigen Verpackungsmaterial (2) ausgerichtet sind und gleichzeitig aktiviert werden, um die gegenüberliegenden Seiten (2a, 2b) eines Verpackungsmaterials (2) gleichzeitig zu bestrahlen, um die gleichzeitige Oberflächensterilisierung beider Seiten des blattförmigen Verpackungsmaterials (2) zu gewährleisten.

9. Einheit nach Anspruch 8, **dadurch gekennzeichnet, dass** die Erzeugungsvorrichtung (20) Elektronenemissionsmittel (27) und Erzeugungsmittel (27, 26) zum Erzeugen eines elektrischen Felds und zum Beschleunigen der Elektronen in einen Strahl enthält, der auf das blattförmige Verpackungsmaterial (2) gerichtet ist.

10. Einheit nach Anspruch 9, **dadurch gekennzeichnet, dass** das elektrische Feld durch eine Spannungsdifferenz von 90 kV oder weniger erzeugt wird.

11. Einheit nach Anspruch 10, **dadurch gekennzeichnet, dass** die Spannungsdifferenz weniger als 80 kV beträgt.

12. Einheit nach einem der Ansprüche 8 - 11, **dadurch gekennzeichnet, dass** diese mindestens einen Schirm (30) enthält, um den Bereich, der die Erzeugungsvorrichtungen (20) zum Erzeugen von Elektronenstrahlen umgibt, abzuschirmen.

13. Einheit nach Anspruch 12, **dadurch gekennzeichnet, dass** der Schirm (30) aus Stahl mit weniger als 20 mm Dicke gefertigt ist.

14. Einheit nach Anspruch 13, **dadurch gekennzeichnet, dass** das Schild (30) aus Blei mit weniger als 3 mm Dicke gefertigt ist.

## Revendications

1. Procédé de stérilisation de matériau d'emballage en feuille (2) pour produire des emballages hermétiques de produits alimentaires fluides, le procédé comprenant l'étape d'irradiation du matériau d'emballage en feuille (2) avec des électrons ; la dite étape d'irradiation comprenant l'étape de direction, sur les faces opposées (2a, 2b) du dit matériau d'emballage en feuille (2), de faisceaux d'électrons à basse tension respectifs, ayant chacun une énergie d'au plus 100 keV, **caractérisé en ce qu'**il comprend l'étape d'alignement mutuel des dits faisceaux d'électrons dans une direction perpendiculaire au dit matériau d'emballage en feuille (2) et de direction des dits faisceaux d'électrons simultanément sur les dites faces opposées (2a, 2b) du dit matériau d'emballage en feuille afin d'assurer la stérilisation simultanée des surfaces des deux côtés du dit matériau d'emballage en feuille (2).

2. Procédé selon la revendication 1 dans lequel la dite étape de direction des faisceaux a pour effet que chaque dit faisceau d'électrons traverse la surface du matériau d'emballage seulement jusqu'à une profondeur de quelques µm pour minimiser toute altération du matériau qui pourrait engendrer un goût déplaisant transmis à un produit alimentaire emballé dans le dit matériau ; le dit matériau d'emballage étant du type ayant une structure multicouche et incluant, sur son côté qui doit venir en contact avec un produit alimentaire, une couche de matière d'arrêt de l'oxygène et de la lumière (12) et une couche de matière plastique thermosoudable (13).

3. Procédé selon la revendication 1 ou 2, **caractérisé en ce que** la dite étape de direction de chaque dit faisceau d'électrons comprend l'étape d'irradiation d'électrons et d'accélération des dits électrons sur les dites faces opposées (2a, 2b) du dit matériau d'emballage en feuille.

4. Procédé selon une quelconque des revendications précédentes, **caractérisé en ce que** la dite étape d'accélération des dits électrons est effectuée par application d'un champ électrique engendré par une différence de potentiel de 90 kV ou moins.

5. Procédé selon la revendication 4, **caractérisé en ce que** la dite différence de potentiel est inférieure à 80 kV.

6. Procédé selon une quelconque des revendications précédentes, **caractérisé par** son application aux faces opposées (2a, 2b) d'un matériau d'emballage en feuille (2) ayant un certain nombre d'éléments auxiliaires pré-montés (5).

7. Procédé selon une quelconque des revendications précédentes, **caractérisé en ce qu'**il est appliqué aux faces opposées (2a, 2b) d'un matériau d'emballage en feuille (2) sous la forme d'une bande continue.

8. Unité (3) de stérilisation d'un matériau d'emballage en feuille (2) pour produire des emballages hermétiques de produits alimentaires fluides, l'unité comprenant un moyen d'irradiation (20) pour irradier le dit matériau d'emballage en feuille (2) avec des électrons, le dit moyen d'irradiation comprenant deux dispositifs de génération (20), pour engendrer des faisceaux d'électrons basse tension, qui sont situés sur des côtés opposés du dit matériau d'emballage en feuille (2) et engendrent des faisceaux d'électrons respectifs dirigés sur les faces opposées (2a, 2b) du matériau d'emballage (2) et ayant chacun une énergie d'au plus 100 keV, **caractérisée en ce que** les dits dispositifs de génération (20) sont mutuellement alignés dans une direction perpendiculaire au dit matériau d'emballage en feuille (2) et simultanément activés, pour irradier simultanément les côtés opposés (2a, 2b) d'un matériau d'emballage (2) afin d'assurer une stérilisation simultanée de surface des deux côtés du dit matériau d'emballage en feuille (2).

9. Unité selon la revendication 8, **caractérisée en ce que** le dit dispositif générateur (20) comprend un moyen d'émission d'électrons (27) et un moyen de génération (27, 26) pour engendrer un champ électrique et accélérer les dits électrons en un faisceau dirigé sur le dit matériau d'emballage en feuille (2).

10. Unité selon la revendication 9, **caractérisée en ce que** le dit champ électrique est engendré par une différence de potentiel de 90 kV ou moins.

11. Unité selon la revendication 10, **caractérisée en ce que** la dite différence de potentiel est inférieure à 80 kV.

12. Unité selon une quelconque des revendications 8 à 11 , **caractérisée en ce qu'**elle comprend au moins un blindage (30) pour protéger la région entourant les dits dispositifs générateurs (20) pour engendrer les faisceaux d'électrons.

13. Unité selon la revendication 12, **caractérisée en ce que** le dit blindage (30) est fabriqué en acier de moins de 20mm d'épaisseur.

14. Unité selon la revendication 13, **caractérisée en ce que** le dit blindage (30) est fabriqué en plomb de 3 mm environ d'épaisseur.
